# EUROPEAN PATENT APPLICATION

(11) **EP 3 598 193 A1**
(43) Date of publication of application: **22.01.2020**
(21) Application number: 19185434.8
(22) Date of filing: 10.07.2019
(51) Int. Cl.: G02B 21/14, G01B 9/02, G02B 21/16, G02B 21/36

(54) **OPTICAL SECTIONING APPARATUS USING ADVANCED OPTICAL INTERFERENCE MICROSCOPY**

(30) Priority: 17.07.2018 CN 201810788288
(71) Applicant: AcuSolutions Inc., Apia (WS)
(72) Inventor: HSU, Kuang-Yu, 103 Taipei City (TW); TSAI, Chien-Chung, 103 Taipei City (TW); JHENG, Dong-Yo, 103 Taipei City (TW)
(74) Representative: Strehl Schübel-Hopf & Partner

(57) **Abstract**

An optical sectioning apparatus including: a first white light source unit for generating a first white light beam; a light mixing unit facing the first white light source unit for dividing the first white light beam into a first partial beam and a second partial beam; a reference end unit for making the second partial beam travel a round trip along an adjustable optical path; a first objective lens having a collimated side facing the light mixing unit; a carrier unit facing a focal side of the first objective lens; a projection lens having a light entering side facing the light mixing unit; and a sensing unit facing a light exiting side of the projection lens.

## Description

### BACKGROUND OF THE INVENTION

### Field of the Invention

The present invention relates to an optical sectioning apparatus, and more particularly to an optical sectioning apparatus using optical interference microscopy, orthogonal polarization spectral absorption microscopy, and fluorescence microscopy.

### Description of the Related Art

In conventional tumor surgery, it takes a lot of time for a pathologist to examine a frozen section to determine if a tumor is cleanly removed. As a result, when the time allowed for the surgery is shorter than the time needed for the pathologist to complete the assessment of intraoperative pathological margin, the tumor cannot be guaranteed to be cleanly removed.

The traditional H&E section can be applied to paraffin sectioning or cryosection, and is still the gold standard for discriminating pathological images in the pathology department. The traditional H&E section uses hematoxylin to color the nuclei bluish violet and uses eosin to color cytoplasm pink. However, during the process of preparing a frozen section of a sample, if the sample contains much water, a formed crystal ice structure will cause a damage to the tissue structure of the sample; and if the sample contains much fat, the fat will still be not frozen at temperatures around -20°C where the other tissues are already frozen, and is therefore easy to slip off from the frozen section to cause an artifact to the frozen section.

OCT (optical coherence tomography) is a newly developed optical imaging technology, which mainly utilizes the phenomena that different tissues of a sample have different responses with regard to light reflection, light absorption, and light scattering and makes use of an optical interference methodology to form an image of the sample (or a live tissue) for diagnosis. Among the OCT technologies, full-field optical coherence tomography (FF-OCT) is the most analytical interferometric microscopy that can observe cell-level interference images non-invasively. However, as the image produced by the FF-OCT does not contain the sub images resulting from excited fluorescent light or from spectral absorption, therefore, when the FF-OCT is applied to a pathological or live tissue, the obtained image will be lacking the details of nuclei and pigmentation.

The US Patent No. 6,104,939 B2, "Method and apparatus for reflected imaging analysis", discloses an imaging device for observing the absorption of visible light spectrum of red blood cells, which is characterized by: using an orthogonal polarization spectral absorption imaging system device, filtering out the background light of the reflection, using the scattering absorption spectrum of hemoglobin and melanin to make a contrast with the background scattering spectrum of white light, and then displaying the spectral absorption plaque of hemoglobin and melanin to further locate the positions and absorption status of red blood cells and melanocytes. The degree of hemoglobin absorption is related to the blood oxygen concentration of red blood cells, and the degree of melanin absorption is related to the degree of skin whiteness.

The US9185357 B2 discloses an optical tissue sectioning apparatus using FF-OCT, which includes a full-field imaging interferometer and an optical sectioning imaging system. This optical tissue sectioning apparatus uses the interferometer to solve the problem resulting from the wide depth of focus by directly deriving the image from the sample, which has a resolution as precise as less than 1 µm both laterally and vertically, getting rid of the need of a fixation process, which includes a freezing procedure, a paraffin-embadded procedure, and a real sectioning procedure.

However, there are two disadvantages in the two mentioned patents:
1. For the US9185357 B2, a shortwave light beam (such as UV light beam) will be attenuated by a beam-splitting film of the beam splitter by 60-90%; and the antireflection film of the beam splitter generally has a low transmission rate for a UV band of wavelengths shorter than 400 nm, resulting in a weak nuclear image.
2. Both the US9185357 B2 and US6104939 B2 can not distinguish the pigment cells and the cell structure of a single sample simultaneously.

As the two disadvantages mentioned above will result in poor image quality and insufficient feature information of the image of the sample, therefore, a novel optical sectioning apparatus is needed.

### SUMMARY OF THE INVENTION

One objective of the present invention is to disclose an optical sectioning apparatus using advanced optical interference microscopy, which can be used not only for imaging a fresh tissue, but also for quickly providing surgical pathology examination information of live skin or tissue under surgery.

Another objective of the present invention is to disclose an optical sectioning apparatus using advanced optical interference microscopy, which utilizes a color mixing unit having a dichroic beam splitter disposed between a first beam splitter and a first objective lens to prevent a shortwave light beam from being attenuated by the first beam splitter when the shortwave light beam travels an optical path to illuminate the sample, thereby increasing a relative strength of a fluorescent signal, shortening an exposure time, and speeding up taking images.

Still another objective of the present invention is to disclose an optical sectioning apparatus using advanced optical interference microscopy, which utilizes a dichroic beam splitter so that when a beam from a sample (the beam including a reflected shortwave beam and a fluorescent beam excited by shortwave illumination) passes through the dichroic beam splitter, the shortwave beam will be filtered out and the fluorescent beam will be passed through, thereby obtaining a fluorescent signal with better contrast, shortening the exposure time, and speeding up taking images.

Still another object of the present invention is to disclose an optical sectioning apparatus using advanced optical interference microscopy, which utilizes a sensor unit having a long pass filter to further filter out a shortwave light beam, thereby increasing the relative strength of a fluorescent signal, shortening an exposure time and speeding up taking images.

Still another objective of the present invention is to disclose an optical sectioning apparatus using advanced optical interference microscopy, which utilizes an optical sectioning scheme characterized in: (1) combining optical interference microscopy with orthogonal polarization spectral absorption image to simultaneously obtain the image of cell structure and the image of pigment areas; (2) combining optical interference microscopy with fluorescence microscopy to obtain the image of cell structure and the image of nuclei successively; and (3) combining optical interference microscopy with orthogonal polarization spectral absorption image to simultaneously obtain the image of cell structure and the image of pigment areas, and then further combining fluorescence microscopy to obtain the image of nuclei.

Still another objective of the present invention is to disclose an optical sectioning apparatus using advanced optical interference microscopy, which utilizes a second white light beam generated by a second white light source to illuminate a H&E frozen section sample or paraffin section sample to obtain a penetration image for comparing with the images obtained by the above schemes.

To attain the foregoing objectives, an optical sectioning apparatus using advanced optical interference microscopy is proposed having: a first white light source unit for generating a first white light beam, the first white light beam having a frequency spectrum including a broad band and a narrow band; a light mixing unit having a first side, a second side, a third side, and a fourth side, the first side facing the first white light source unit, and capable of dividing the first white light beam incident on the first side into a first partial beam passing through the second side and a second partial beam passing through the third side; a reference end unit for making the second partial beam travel a round trip along an adjustable optical path and then return to the light mixing unit; a first objective lens having a collimated side and a focal side, the collimated side facing the second side of the light mixing unit; a carrier unit for carrying a live tissue or a sample dyed with a fluorescent agent, the carrier unit facing the focal side of the first objective lens; a projection lens having a light entering side and a light exiting side, the light entering side facing the fourth side of the light mixing unit; and a sensing unit facing the light exiting side of the projection lens.

In one embodiment, the light mixing unit includes a first polarizer and a first beam splitter, the first beam splitter having a first side, a second side, a third side and a fourth side, the first side of the first beam splitter facing the first polarizer, the first polarizer also facing the first side of the light mixing unit, the second side of the first beam splitter facing the second side of the light mixing unit, the third side of the first beam splitter facing the third side of the light mixing unit, and the fourth side of the first beam splitter facing the fourth side of the light mixing unit; or the light mixing unit includes a first polarizer, a first beam splitter, a shortwave light source unit and a dichroic beam splitter, the first beam splitter having a first side, a second side, a third side and a fourth side, the first side of the first beam splitter facing the first polarizer, the first polarizer also facing the first side of the light mixing unit, the third side of the first beam splitter facing the third side of the light mixing unit, the fourth side of the first beam splitter facing the fourth side of the light mixing unit, the shortwave light source unit being configured to generate a shortwave beam, the dichroic beam splitter having a first a side, a second side and a third side, the first side facing the shortwave source device, the second side facing the second side of the light mixing unit, the third side facing the second side of the first beam splitter, and the dichroic beam splitter being configured to make a light beam having a wavelength shorter than a predetermined wavelength unable to penetrate but reflected to the collimated side of the first objective lens, and the wavelength of the shortwave beam being less than the predetermined wavelength.

In one embodiment, the reference end unit includes: an optical path delay unit having a first side and a second side, the first side facing the third side of the light mixing unit; a second objective lens having a collimated side and a focal side, the collimated side facing the second side of the optical path delay unit; and a reflective mirror facing the focal side of the second objective lens for reflecting the light beam emitted from the focal side, wherein the optical path delay unit is configured to adjust the adjustable optical path to make the adjustable optical path symmetric to a sample optical path formed by the carrier unit, the first objective lens, and the light mixing unit.

In one embodiment, both the first white light source unit and the shortwave light source unit include: a light source; or a light source and a slit; or a light source and a grating; or a light source, a grating and a flip mirror with adjustable tilt angles; or a light source, a slit and a flip mirror with adjustable tilt angles; or a strip-shaped LED light source.

In one embodiment, the sensing unit includes a second beam splitter, a second polarizer, a third polarizer, a long pass filter, a monochromatic two-dimensional photosensitive device, and a color two-dimensional photosensitive device, the second beam splitter having a first side, a second side, and a third side, the first side facing the projection lens, the third side outputting the first white light beam to pass through the third polarizer and then illuminate the color two-dimensional photosensitive device to form a color image, and the second side outputting a fluorescent light beam and the first white light beam having the broadband frequency band and the narrow frequency band to pass through the second polarizer and the long pass filter to filter out the narrow frequency band and then illuminate the monochromatic two-dimensional photosensitive device to form a monochromatic image, wherein a first quarter wave plate is disposed near the second side of the first beam splitter of the light mixing unit, and a second quarter wave plate is disposed near the third side of the first beam splitter of the light mixing unit, the first polarizing device having a first polarization direction, the second polarizer having a second polarization direction, the third polarizer having a third polarization direction, the second polarization direction and the third polarization direction being perpendicular to each other, the first polarization direction and the second polarization direction being perpendicular to each other, the first quarter wave plate having a first optical axis direction, the second quarter wave plate having a second optical axis direction, wherein the first optical axis direction and the second optical axis direction are both disposed between the first polarization direction and the second polarization direction for enhancing an interference efficiency and an image quality of an orthogonal polarization spectral absorption image.

In one embodiment, the first white light beam has a wavelength ranging from 400 nm to 800 nm and includes a blue narrowband having a wavelength ranging from 400 nm to 480 nm and a yellow or orange broadband having a wavelength ranging from 480 nm to 800 nm, the shortwave light beam has a wavelength ranging from 350 nm to 410 nm, the first quarter wave plate, the second quarter wave plate, the first beam splitter, the second beam splitter, the first polarizer, the second polarizer and the third polarizer are configured to operate with a wavelength ranging from 400 nm to 800 nm, the dichroic beam splitter has a cut-off wavelength range between 360 nm and 420 nm, and the long pass filter has a cut-off wavelength range between 460 nm and 510 nm.

In one embodiment, the sensing unit includes a polarization beam splitter, a long pass filter, a monochromatic two-dimensional photosensitive device, and a color two-dimensional photosensitive device, and the polarization beam splitter having a first side, a second side, and a third side, the first side facing the projection lens, the third side outputting the first white light beam to illuminate the color two-dimensional photosensitive device to form a color image, and the second side outputting a fluorescent light beam and the first white light beam having the broadband frequency band and the narrow frequency band to pass through the long pass filter to filter out the narrow frequency band and then illuminate the monochromatic two-dimensional photosensitive device to form a monochromatic image, wherein a first quarter wave plate is disposed near the second side of the first beam splitter of the light mixing unit, and a second quarter wave plate is disposed near the third side of the first beam splitter of the light mixing unit, the first polarizing device having a first polarization direction, the polarization beam splitter having a fourth polarization direction (S-polarization), the first polarization direction and the fourth polarization direction being perpendicular to each other, the first quarter wave plate having a first optical axis direction, the second quarter wave plate having a second optical axis direction, wherein the first optical axis direction and the second optical axis direction are both disposed between the first polarization direction and the fourth polarization direction for enhancing an interference efficiency and an image quality of an orthogonal polarization spectral absorption image.

In one embodiment, the first white light beam has a wavelength ranging from 400 nm to 800 nm and includes a blue narrowband having a wavelength ranging from 400 nm to 480 nm and a yellow or orange broadband having a wavelength ranging from 480 nm to 800 nm, the shortwave light beam having a wavelength ranging from 350 nm to 410 nm, the first quarter wave plate, the second quarter wave plate, the first beam splitter, the second beam splitter, the first polarizer, and the polarization beam splitter being configured to operate with a wavelength ranging from 400 nm to 800 nm, the dichroic beam splitter having a cut-off wavelength range between 360 nm and 420 nm, and the long pass filter having a cut-off wavelength range between 460 nm and 510 nm.

In one embodiment, the optical sectioning apparatus further includes an information processing unit for executing an image processing procedure.

In one embodiment, the reference end unit further has an axial motion platform, and the carrier unit further includes a 3-D motion platform, so that by using the axial motion platform to move the second objective lens and the reflective mirror of the reference arm unit, using the 3-D motion platform to move the sample dyed with a fluorescent agent, and adjusting the optical path delay unit, the information processing apparatus can derive a 3-D image of the sample accordingly.

In one embodiment, the carrier unit further has a 3-D motion platform and a second white light source unit, wherein the second white light source unit includes a white LED, a white halogen lamp, or a tungsten lamp, and the second white light source unit is configured to provide the first objective lens with a suitable penetration brightness, so that when a H&E slice is placed on the carrier unit, the 3-D motion platform will move the H&E slice and the second white light source will generate a second white light beam to illuminate the H&E slice to enable the information processing unit to execute the image processing procedure according to image data sensed by the sensing unit to generate an H&E image.

To make it easier for our examiner to understand the objective of the invention, its structure, innovative features, and performance, we use preferred embodiments together with the accompanying drawings for the detailed description of the invention.

### BRIEF DESCRIPTION OF THE DRAWINGS

FIG.1a illustrates a block diagram of an optical sectioning apparatus using advanced optical interference microscopy according to one embodiment of the present invention.
FIG. 1b is a schematic view showing the operations of beam splitting and focusing of the optical sectioning apparatus of FIG.1a.
FIG.2 illustrates a block diagram of the reference end unit of FIG. 1 according to one embodiment of the present invention.
FIG.3a illustrates a block diagram of the light mixing unit of FIG. 1a according to one embodiment of the present invention.
FIG.3b illustrates a block diagram of the light mixing unit of FIG. 1a according to another embodiment of the present invention.
FIG.3c illustrates a block diagram of the light mixing unit of FIG. 1a according to another embodiment of the present invention.
FIG.3d illustrates a block diagram of the light mixing unit of FIG. 1a according to another embodiment of the present invention.
FIG. 4 is a schematic view showing the operations of light collection and projection of the optical sectioning apparatus of FIG.1a.
FIG.5a illustrates a block diagram of the sensing unit of FIG. 1a according to one embodiment of the present invention.
FIG.5b illustrates a block diagram of the sensing unit of FIG. 1a according to another embodiment of the present invention.
FIG.6 illustrates a block diagram of an optical sectioning apparatus using advanced optical interference microscopy according to another embodiment of the present invention.

### DETAILED DESCRIPTION OF THE PREFERRED EMBODIMENTS

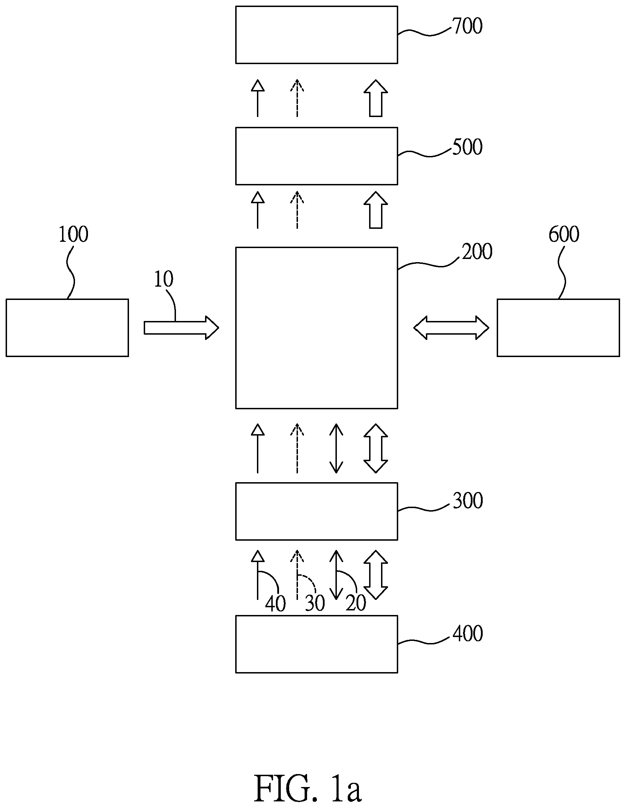

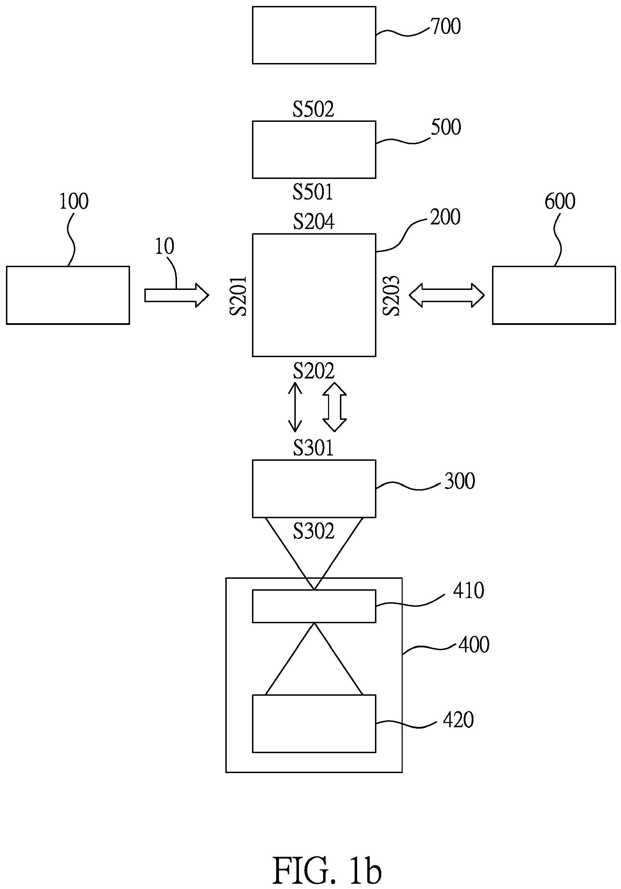

Please refer to FIG. 1a-b, where FIG.1a illustrates a block diagram of an optical sectioning apparatus using advanced optical interference microscopy according to one embodiment of the present invention; FIG. 1b is a schematic view showing the operations of beam splitting and focusing of the optical sectioning apparatus of FIG.1a.

As shown in FIG. 1a, the optical sectioning apparatus using advanced optical interference microscopy of the present invention includes a first white light source unit 100, a light mixing unit 200, a first objective lens 300, a carrier unit 400 for carrying a live tissue or a sample, a projection lens 500, a reference end unit 600 and a sensing unit 700.

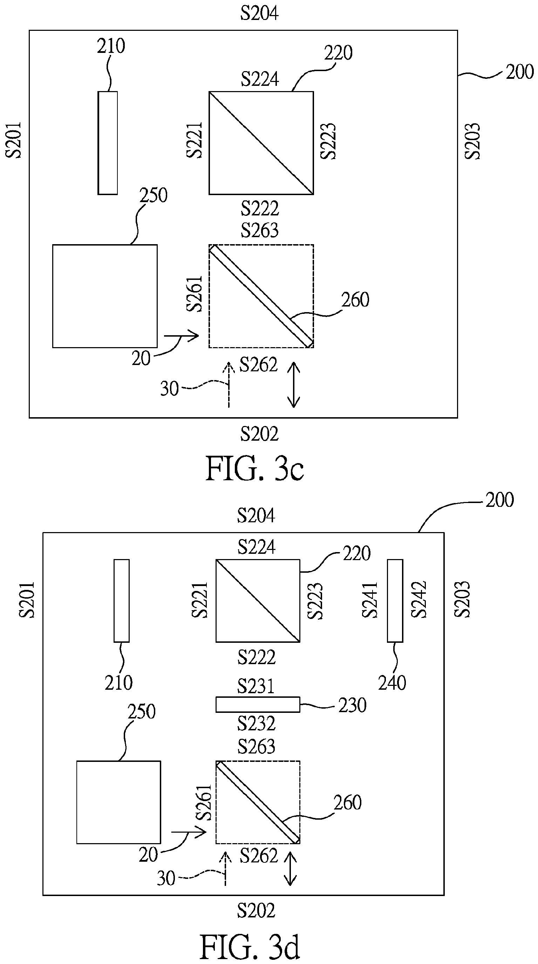

The first white light source unit 100 is configured to generate a first white light beam 10, the travel direction of the first white light beam 10 being indicated by a hollow head arrow; the light mixing unit 200 includes a shortwave light source unit 250 (referring to FIG. 3c and FIG. 3d, which is not shown in FIG. la), the shortwave light source unit 250 is configured to generate a shortwave beam 20, and the traveling direction of the shortwave beam 20 is indicated by a solid arrow; the carrying unit 400 is for carrying a sample 410 dyed with a fluorescent agent (referring to FIG. 1b, which is not shown in FIG. 1a), the fluorescent agent will release a fluorescent beam 30 when irradiated by the shortwave beam 20, and the travel direction of the fluorescent beam 30 is indicated by a dashed arrow.

As shown in FIG. 1b, the first white light source unit 100 is configured to generate a first white light beam 10, the light mixing unit 200 has a first side S201, a second side S202, a third side S203 and a fourth side S204, the first side S201 facing the first white light source unit 100, and the light mixing unit 200 divides the first white light beam 10 incident on the first side S201 into a first partial beam passing through the second side S202 and a second partial beam passing through the third side S203.

The reference end unit 600 is configured to make the second partial beam travel a round trip along an adjustable optical path and then return to the light mixing unit 200; the first objective lens 300 has a collimated side S301 and a focal side S302, the collimated side S301 facing the second side S202 of the light mixing unit 200; the carrier unit 400 faces the focal side S302 of the first objective lens 300; the projection lens 500 has a light entering side S501 and a light exiting side S502, the light entering side S501 facing the fourth side S204 of the light mixing unit 200; and the sensing unit 700 faces the light exiting side S502 of the projection lens 500.

Besides, the carrier unit 400, the first objective lens 300 and the light mixing unit 200 form a sample optical path.

The first white light beam 10 can have a frequency spectrum including a wide band and a narrow band; both the first white light source unit 100 and the shortwave light source unit 250 (not shown in the figure) can include, for example but not limited to: a light source; or a light source and a slit; or a light source and a grating; or a light source, a grating and a flip mirror with adjustable tilt angles; or a light source, a slit and a flip mirror with adjustable tilt angles; or a strip-shaped LED light source. As the mentioned embodiments of the shortwave light source unit 250 are all conventional techniques, they will not be further addressed.

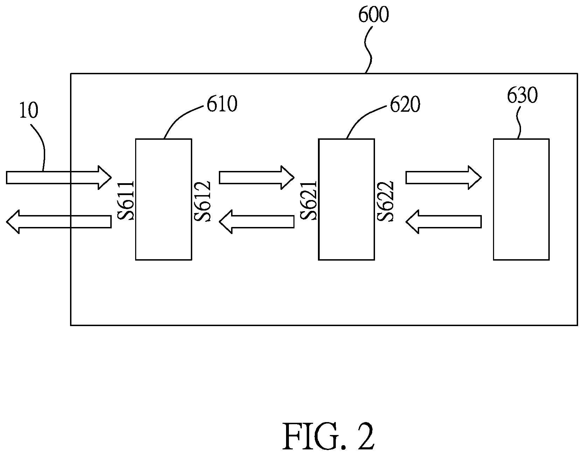

Please refer to FIG. 2, which illustrates a block diagram of the reference end unit of FIG. 1 according to one embodiment of the present invention.

As shown in FIG. 2, the reference end unit 600 includes: an optical path delay unit 610; a second objective lens 620 and a reflective mirror 630.

The optical path delay unit 610 has a first side S611 and a second side S612, the first side S611 facing the third side S203 of the light mixing unit 200 (not shown in the figure); the second objective lens 620 has a collimated side S621 and a focal side S622, the collimated side S621 facing the second side S612 of the optical path delay unit 610; and the reflective mirror 630 faces the focal side S622 of the second objective lens 620 for reflecting the light beam emitted from the focal side S622.

The light mixing unit 200 (shown in FIG. 1b), the optical path delay unit 610, the second objective lens 620, and the reflective mirror 630 form an adjustable optical path. The reference end unit 600 is configured to make the second partial beam travel a round trip along the adjustable optical path and then return to the light mixing unit 200, where the optical path delay unit 610 is configured to adjust the adjustable optical path to make the adjustable optical path symmetric to a sample optical path formed by the carrier unit 400, the first objective lens 300, and the light mixing unit 200.

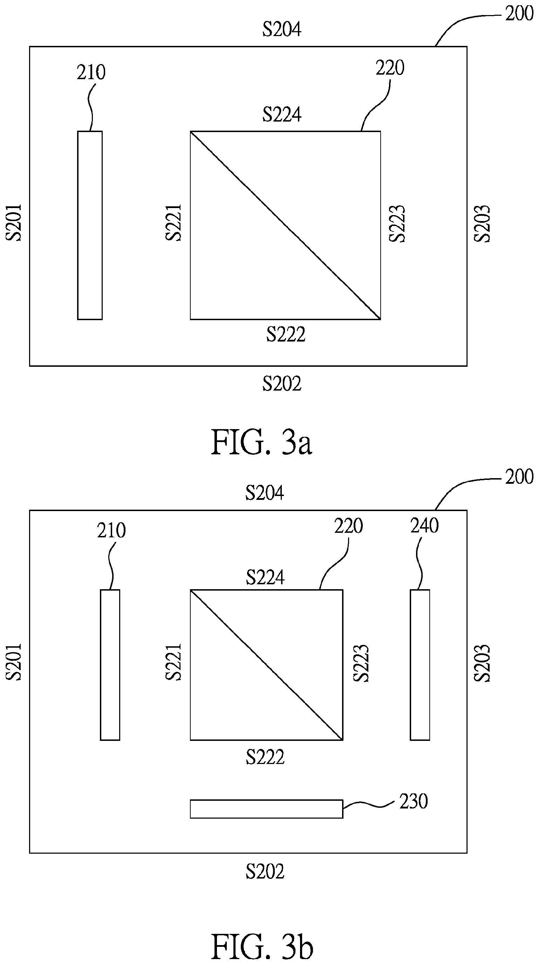

Please refer to FIG. 3a-b, where FIG.3a illustrates a block diagram of the light mixing unit of FIG. 1a according to one embodiment of the present invention; and FIG.3b illustrates a block diagram of the light mixing unit of FIG. 1a according to another embodiment of the present invention.

As shown in FIG. 3a, the light mixing unit 200 includes a first polarizer 210 and a first beam splitter 220.

The first beam splitter 220 has a first side S221, a second side S222, a third side S223 and a fourth side S224. The first side S221 faces the first polarizer 210, the first polarizer 210 faces the first side S201 of the light mixing unit 200, the second side S222 faces the second side S202 of the light mixing unit 200, the third side S223 faces the third side S203 of the light mixing unit 200, and the fourth side S224 faces the fourth side S204 of the light mixing unit 200.

As shown in FIG. 3b, the second side S222 of the first beam splitter 220 further has a first quarter wave plate 230 facing the second side S202 of the light mixing unit 200. The third side S223 of the first beam splitter 220 further has a second quarter wave plate 240 facing the third side S203 of the light mixing unit 200.

Please refer to FIG. 3c-d, where FIG.3c illustrates a block diagram of the light mixing unit of FIG. 1a according to another embodiment of the present invention; and FIG.3d illustrates a block diagram of the light mixing unit of FIG. 1a according to still another embodiment of the present invention.

As shown in FIG. 3c, the light mixing unit 200 includes a first polarizer 210, a first beam splitter 220, a shortwave light source unit 250, and a dichroic beam splitter 260.

The first beam splitter 220 has a first side S221, a second side S222, a third side S223 and a fourth side S224. The first side S221 of the first beam splitter 220 faces the first polarizer 210, the first polarizer 210 faces the first side S201 of the light mixing unit 200, the third side S223 of the first beam splitter 220 faces the third side S203 of the light mixing unit 200, and the fourth side S224 of the first beam splitter 220 faces the fourth side S204 of the light mixing unit 200. The shortwave light source unit 250 is configured to generate a shortwave beam 20, the dichroic beam splitter 260 has a first side S261, a second side S262 and a third side S263. The first side S261 faces the shortwave light source device 250, the second side S262 faces the second side S202 of the light mixing unit 200, and the third side S263 faces the second side S222 of the first beam splitter 220. The dichroic beam splitter 260 is configured to make a light beam having a wavelength shorter than a predetermined wavelength unable to penetrate but instead reflected to the collimated side S301 of the first objective lens 300 and converged at the focal side S301, and the wavelength of the shortwave beam is less than the predetermined wavelength.

As shown in FIG. 3d, the second side S222 of the first beam splitter 220 further has a first quarter wave plate 230 facing the third side S263 of the dichroic beam splitter 260. The third side S263 further includes a second quarter wave plate 240 facing the third side S203 of the light mixing unit 200.

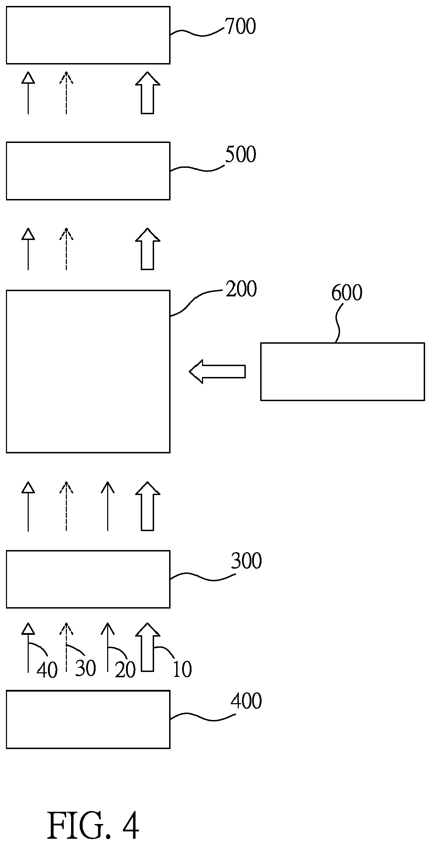

Please refer to FIG. 4, which is a schematic view showing the operations of light collection and projection of the optical sectioning apparatus of FIG.1a.

As shown in the figure, the carrier unit 400 can absorb the shortwave beam 20 and reflect the first partial beam of the first white light beam 10, and the sample 410 dyed with a fluorescent agent (not shown in the figure) emits a fluorescent beam 30 when illuminated by the shortwave beam 20.

The dichroic beam splitter 260 (not shown in the figure) is configured to make a light beam having a wavelength shorter than a predetermined wavelength unable to penetrate but reflected to the collimated side S301 of the first objective lens 300 (not shown in the figure), and only the first partial beam of the first white light beam 10 and the fluorescent beam 30 can penetrate the dichroic beam splitter 260.

The reference end unit 600 is configured to make the second partial beam of the first white light beam 10 travel a round trip along an adjustable optical path and then return to the light mixing unit 200; the third side S203 of the light mixing unit 200 (not shown in the figure) faces the reference end unit 600 to receive the second partial beam of the first white light beam 10 returned from the reference unit 600, and the second partial beam is then guided out of S204 (not shown in the figure) to illuminate the projection lens 500. The light mixing unit 200 faces the first objective lens 300 with the second side S202 (not shown in the figure) for guiding the fluorescent light beam 30 and the first partial beam of the first white light beam 10 to the projection lens 500 via S204 (not shown in the figure).

As the adjustable optical path and the sample optical path are symmetric to each other (that is, the length difference of the adjustable optical path and the sample optical path is in between a coherent length), a broadband light beam of the second partial beam reflected by the adjustable optical path and a broadband light beam of the first partial beam reflected by the sample optical path combines to produce an optical interference, which is a conventional technique and will not be further addressed.

The projection lens 500 has a light entering side S501 (not shown in the figure) and a light exiting side S502 (not shown in the figure), and the light entering side S501 faces the fourth side S204 (not shown in the figure) of the light mixing unit 200. The light exiting side S502 is used to project the first white light beam 10 and the fluorescent light beam 30 to the sensing unit 700.

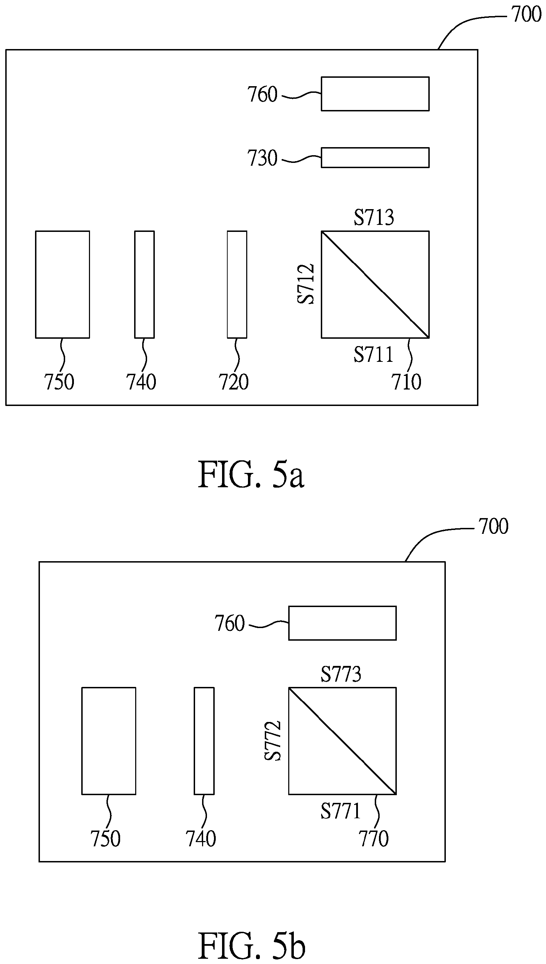

Please refer to FIG. 5a, which illustrates a block diagram of the sensing unit of FIG. 1a according to one embodiment of the present invention.

As shown in the figure, the sensing unit includes a second beam splitter 710, a second polarizer 720, a third polarizer 730, a long pass filter 740, a monochromatic two-dimensional photosensitive device 750 and a color two-dimensional photosensitive device 760.

The second beam splitter 710 has a first side S711, a second side S712 and a third side S713. The first side S711 faces the projection lens 500 and receives its projection beam. The first white light beam 10 is split by the second beam splitter 710, guided out of the third side S713 to be polarized and absorbed by the third polarizer 730, and then projected on the color two-dimensional photosensitive device 760 to form a color image; both a fluorescent light beam 30 and a first white light beam 10 having the broad band and the narrow band are split by the second beam splitter 710, guided out of the second side S712 to be polarized and absorbed by the second polarizer 720, processed by the long pass filter 740 to filter out the narrow frequency band, and then projected on the monochromatic two-dimensional photosensitive device 750 to form a monochromatic image.

The first polarizer 210 (shown in FIG. 3b) has a first polarization direction, the second polarizer 720 has a second polarization direction, and the third polarizer 730 has a third polarization direction. The second polarization direction and the third polarization direction are perpendicular to each other, the first polarization direction and the second polarization direction are perpendicular to each other, and the first quarter wave plate 230 has a first optical axis direction, the second quarter wave plate 240 has a second optical axis direction, where the first optical axis direction and the second optical axis direction are both disposed between the first polarization direction and the second polarization direction for enhancing an interference efficiency and an image quality of an orthogonal polarization spectral absorption image.

In one embodiment, the first white light beam 10 has a wavelength ranging from 400 nm to 800 nm and includes a blue narrowband light having a wavelength ranging from 400 nm to 480 nm and a yellow or orange broadband having a wavelength ranging from 480 nm to 800 nm, the shortwave light beam 20 has a wavelength ranging from 350 nm to 410 nm, the first quarter wave plate 230, the second quarter wave plate 240, the first beam splitter 220, the second beam splitter 710, the second polarizer 720 and the third polarizer 730 are configured to operate with a wavelength ranging from 400 nm to 800 nm, the dichroic beam splitter 260 has a cut-off wavelength range between 360 nm and 420 nm, and the long pass filter 740 has a cut-off wavelength range between 460 nm and 510 nm.

Please refer to FIG. 5b, which is a block diagram of another embodiment of the sensing unit of FIG. 1a. As shown in the figure, the sensing unit includes a polarization beam splitter 770, a long pass filter 740, a monochromatic two-dimensional photosensitive device 750 and a color two-dimensional photosensitive device 760.

The polarization beam splitter 770 has a first side S771, a second side S772 and a third side S773, the first side S771 facing the projection lens 500 and receiving its projection beam. The first white light beam 10 is polarized and split by the polarization beam splitter 770, guided out of the third side S773, and then projected on the color two-dimensional photosensitive device 760 to form a color image; both a fluorescent light beam 30 and a first white light beam 10 having the broad band and the narrow band are polarized and split by the polarization beam splitter 770, guided out of the second side S772 to be processed by the long pass filter 740 to filter out the narrow frequency band, and then projected on the monochromatic two-dimensional photosensitive device 750 to form a monochromatic image.

The first polarizing device 210 (referring to FIG. 3b) has a first polarization direction, the polarization beam splitter 770 has a fourth polarization direction (S-polarization), the first polarization direction and the fourth polarization direction being perpendicular to each other, the first quarter wave plate 230 having a first optical axis direction, the second quarter wave plate 240 having a second optical axis direction, where the first optical axis direction and the second optical axis direction are both disposed between the first polarization direction and the fourth polarization direction for enhancing an interference efficiency and an image quality of an orthogonal polarization spectral absorption image.

In one embodiment, the first white light beam 10 has a wavelength ranging from 400 nm to 800 nm and includes a blue narrowband light having a wavelength ranging from 400 nm to 480 nm and a yellow or orange broadband having a wavelength ranging from 480 nm to 800 nm, the shortwave light beam 20 has a wavelength ranging from 350 nm to 410 nm, the first quarter wave plate 230, the second quarter wave plate 240, the first beam splitter 220, the second beam splitter 710, the first polarizer 210, and the polarization beam splitter 770 are configured to operate with a wavelength ranging from 400 nm to 800 nm, the dichroic beam splitter 260 has a cut-off wavelength range between 360 nm and 420 nm, and the long pass filter 740 has a cut-off wavelength range between 460 nm and 510 nm.

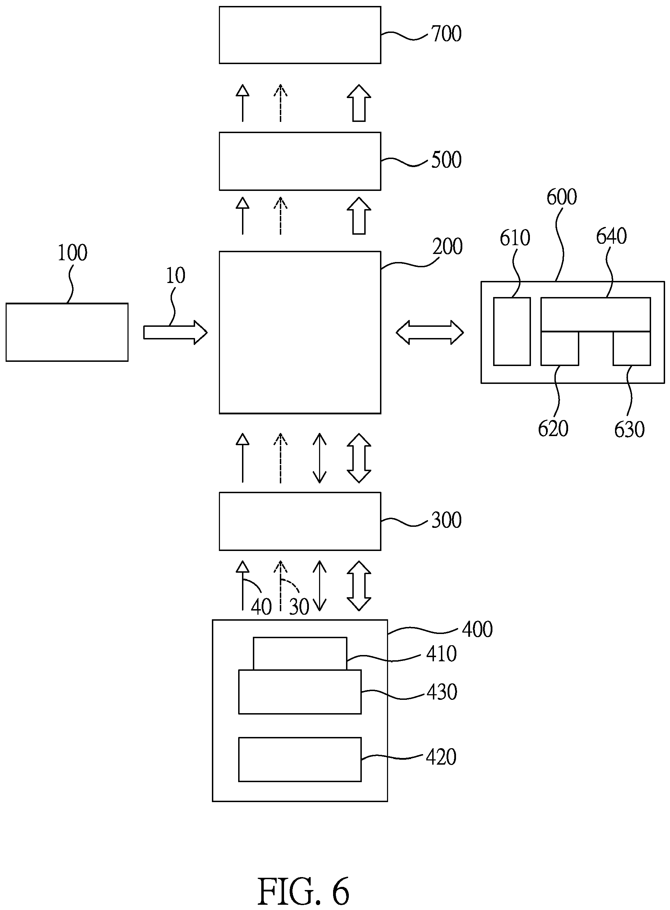

Please refer to FIG. 6, which illustrates a block diagram of an optical sectioning apparatus using advanced optical interference microscopy according to another embodiment of the present invention.

As shown in the figure, the reference end unit 600 further has an axial motion platform 640, and the carrier unit 400 further includes a 3-D motion platform 430. Besides, the optical sectioning apparatus of the present invention further includes an information processing apparatus (not shown in the figure) for executing an image processing procedure.

By using the axial motion platform 640 to move the second objective lens 620 and the reflective mirror 630, using the 3-D motion platform 430 to move the sample 410 dyed with a fluorescent agent, and adjusting the optical path delay unit 610, the information processing apparatus can derive a 3-D image (not shown) of the sample accordingly. As the 3-D image forming method is a conventional technology, it will not be further addressed.

In addition, the sample carrying unit 400 further has a second white light source unit 420, where the second white light source unit 420 includes a white LED, a white halogen lamp, or a tungsten lamp, and the second white light source unit 420 is configured to provide the first objective lens 300 with a suitable penetration brightness, so that when a H&E slice is placed on the carrier unit 400, the 3-D motion platform 430 will move the H&E slice and the second white light source 420 will generate a second white light beam 40 to illuminate the H&E slice, so as to enable the information processing unit to execute the image processing procedure according to image data sensed by the sensing unit to generate an H&E image.

With the above disclosed designs, the present invention has the following advantages:
1. The optical sectioning apparatus using advanced optical interference microscopy of the present invention can be used not only for imaging a fresh tissue, but also for quickly providing surgical pathology examination information of live skin or tissue under surgery.
2. The optical sectioning apparatus using advanced optical interference microscopy of the present invention utilizes a color mixing unit having a dichroic beam splitter disposed between a first beam splitter and a first objective lens to prevent a shortwave light beam from being attenuated by the first beam splitter when the shortwave light beam travels an optical path to illuminate the sample, thereby increasing a relative strength of a fluorescent signal, shortening an exposure time, and speeding up taking images.
3. The optical sectioning apparatus using advanced optical interference microscopy of the present invention utilizes a dichroic beam splitter so that when a beam from a sample (the beam including a reflected shortwave beam and a fluorescent beam excited by shortwave illumination) passes through the dichroic beam splitter, the shortwave beam will be filtered out and the fluorescent beam will be passed through, thereby obtaining a fluorescent signal with better contrast, shortening the exposure time, and speeding up taking images.
4. The optical sectioning apparatus using advanced optical interference microscopy of the present invention utilizes a sensor unit having a long pass filter to further filter out a shortwave light beam, thereby increasing the relative strength of a fluorescent signal, shortening an exposure time and speeding up taking images.
5. The optical sectioning apparatus using advanced optical interference microscopy of the present invention utilizes an optical sectioning scheme characterized in: (1) combining optical interference microscopy with orthogonal polarization spectral absorption image to simultaneously obtain the image of cell structure and the image of pigment areas; (2) combining optical interference microscopy with fluorescence microscopy to obtain the image of cell structure and the image of nuclei successively; and (3) combining optical interference microscopy with orthogonal polarization spectral absorption image to simultaneously obtain the image of cell structure and the image of pigment areas, and then further combining fluorescence microscopy to obtain the image of nuclei.
6. The optical sectioning apparatus using advanced optical interference microscopy of the present invention utilizes a second white light beam generated by a second white light source to illuminate a H&E frozen section sample or paraffin section sample to obtain a penetration image for comparing with the images obtained by the above schemes.

While the invention has been described by way of example and in terms of preferred embodiments, it is to be understood that the invention is not limited thereto. On the contrary, it is intended to cover various modifications and similar arrangements and procedures, and the scope of the appended claims therefore should be accorded the broadest interpretation so as to encompass all such modifications and similar arrangements and procedures.

In summation of the above description, the present invention herein enhances the performance over the conventional structure and further complies with the patent application requirements and is submitted to the Patent and Trademark Office for review and granting of the commensurate patent rights.

## Claims

1. An optical sectioning apparatus using advanced optical interference microscopy, comprising:
a first white light source unit (100) for generating a first white light beam (10), the first white light beam (10) having a frequency spectrum including a broad band and a narrow band;
a light mixing unit (200) having a first side (S201), a second side (S202), a third side (S203), and a fourth side (S204), the first side (S201) facing the first white light source unit (100), and capable of dividing the first white light beam (10) incident on the first side (S201) into a first partial beam passing through the second side (S202) and a second partial beam passing through the third side (S203);
a reference end unit (600) for making the second partial beam travel a round trip along an adjustable optical path and then return to the light mixing unit (200);
a first objective lens (300) having a collimated side (S301) and a focal side (S302), the collimated side (S301) facing the second side (S202) of the light mixing unit (200);
a carrier unit (400) for carrying a live tissue or a sample (410) dyed with a fluorescent agent, the carrier unit (400) facing the focal side (S302) of the first objective lens (300);
a projection lens (500) having a light entering side (S501) and a light exiting side (S502), the light entering side (S501) facing the fourth side (S204) of the light mixing unit (200); and
a sensing unit (700) facing the light exiting side (S502) of the projection lens (500).

2. The optical sectioning apparatus using advanced optical interference microscopy according to claim 1, wherein the light mixing unit (200) comprises a first polarizer (210) and a first beam splitter (220), the first beam splitter (220) having a first side (S221), a second side (S222), a third side (S223) and a fourth side (S224), the first side (S221) of the first beam splitter (220) facing the first polarizer (210), the first polarizer (210) also facing the first side (S201) of the light mixing unit (200), the second side (S222) of the first beam splitter (220) facing the second side (S202) of the light mixing unit (200), the third side (S223) of the first beam splitter (220) facing the third side (S203) of the light mixing unit (200), and the fourth side (S224) of the first beam splitter (220) facing the fourth side (S204) of the light mixing unit (200); or the light mixing unit (200) includes a first polarizer (210), a first beam splitter (220), a shortwave light source unit (250) and a dichroic beam splitter (260), the first beam splitter (220) having a first side (S221), a second side (S222), a third side (S223) and a fourth side (S224), the first side (S221) of the first beam splitter (220) facing the first polarizer (210), the first polarizer (210) also facing the first side (S201) of the light mixing unit (200), the third side (S223) of the first beam splitter (220) facing the third side (S203) of the light mixing unit (200), the fourth side (S224) of the first beam splitter (200) facing the fourth side (S204) of the light mixing unit (200), the shortwave light source unit (250) being configured to generate a shortwave beam (20), the dichroic beam splitter (260) having a first a side (S261), a second side (S262) and a third side (S263), the first side (S261) facing the shortwave source device (250), the second side (S262) facing the second side (S202) of the light mixing unit (200), the third side (S263) facing the second side (S222) of the first beam splitter (220), and the dichroic beam splitter (260) being configured to make a light beam having a wavelength shorter than a predetermined wavelength unable to penetrate but reflected to the collimated side (S301) of the first objective lens (300), and the wavelength of the shortwave beam being less than the predetermined wavelength.

3. The optical sectioning apparatus using advanced optical interference microscopy according to claim 1 or 2, wherein the reference end unit (600) comprises:
an optical path delay unit (610) having a first side (S611) and a second side (S612), the first side (S611) facing the third side (S203) of the light mixing unit (200);
a second objective lens (620) having a collimated side (S621) and a focal side (S622), the collimated side (S621) facing the second side (S612) of the optical path delay unit (610); and
a reflective mirror (630) facing the focal side (S622) of the second objective lens (620) for reflecting the light beam emitted from the focal side (S622), wherein the optical path delay unit (610) is configured to adjust the adjustable optical path to make the adjustable optical path symmetric to a sample optical path formed by the carrier unit (400), the first objective lens (300), and the light mixing unit (200).

4. The optical sectioning apparatus using advanced optical interference microscopy according to claim 1 or 2, wherein both the first white light source unit (100) and the shortwave light source unit (250) include: a light source; or a light source and a slit; or a light source and a grating; or a light source, a grating and a flip mirror with adjustable tilt angles; or a light source, a slit and a flip mirror with adjustable tilt angles; or a strip-shaped LED light source.

5. The optical sectioning apparatus using advanced optical interference microscopy according to claim 2, wherein the sensing unit (700) comprises a second beam splitter (710), a second polarizer (720), a third polarizer (730), a long pass filter (740), a monochromatic two-dimensional photosensitive device (750) and a color two-dimensional photosensitive device (760), the second beam splitter (710) having a first side (S711), a second side (S712), and a third side (S713), the first side (S711) facing the projection lens (500), the third side (S713) outputting the first white light beam (10) to pass through the third polarizer (730) and then illuminate the color two-dimensional photosensitive device (760) to form a color image, and the second side (S712) outputting a fluorescent light beam (30) and the first white light beam (10) having the broadband frequency band and the narrow frequency band to pass through the second polarizer (720) and the long pass filter (740) to filter out the narrow frequency band and then illuminate the monochromatic two-dimensional photosensitive device (750) to form a monochromatic image, wherein a first quarter wave plate (230) is disposed near the second side (S222) of the first beam splitter (220) of the light mixing unit (200), and a second quarter wave plate (240) is disposed near the third side (S223) of the first beam splitter (220) of the light mixing unit (200), the first polarizing device (210) having a first polarization direction, the second polarizer (720) having a second polarization direction, the third polarizer (730) having a third polarization direction, the second polarization direction and the third polarization direction being perpendicular to each other, the first polarization direction and the second polarization direction being perpendicular to each other, the first quarter wave plate (230) having a first optical axis direction, the second quarter wave plate (240) having a second optical axis direction, wherein the first optical axis direction and the second optical axis direction are both disposed between the first polarization direction and the second polarization direction for enhancing an interference efficiency and an image quality of an orthogonal polarization spectral absorption image.

6. The optical sectioning apparatus using advanced optical interference microscopy according to claim 5, wherein the first white light beam (10) has a wavelength ranging from 400 nm to 800 nm and includes a blue narrowband having a wavelength ranging from 400 nm to 480 nm and a yellow or orange broadband having a wavelength ranging from 480 nm to 800 nm, the shortwave light beam (20) has a wavelength ranging from 350 nm to 410 nm, the first quarter wave plate (230), the second quarter wave plate (240), the first beam splitter (220), the second beam splitter (710), the first polarizer (210), the second polarizer (720) and the third polarizer (730) are configured to operate with a wavelength ranging from 400 nm to 800 nm, the dichroic beam splitter (260) has a cut-off wavelength range between 360 nm and 420 nm, and the long pass filter (740) has a cut-off wavelength range between 460 nm and 510 nm.

7. The optical sectioning apparatus using advanced optical interference microscopy according to claim 2, wherein the sensing unit (700) comprises a polarization beam splitter (770), a long pass filter (740), a monochromatic two-dimensional photosensitive device (750), and a color two-dimensional photosensitive device (760), and the polarization beam splitter (770) having a first side (S771), a second side (S772), and a third side (S773), the first side (S771) facing the projection lens (500), the third side (S773) outputting the first white light beam (10) to illuminate the color two-dimensional photosensitive device (760) to form a color image, and the second side (S772) outputting a fluorescent light beam (30) and the first white light beam (10) having the broadband frequency band and the narrow frequency band to pass through the long pass filter (740) to filter out the narrow frequency band and then illuminate the monochromatic two-dimensional photosensitive device (750) to form a monochromatic image, wherein a first quarter wave plate (230) is disposed near the second side (S222) of the first beam splitter (220) of the light mixing unit (200), and a second quarter wave plate (240) is disposed near the third side (S223) of the first beam splitter (220) of the light mixing unit (200), the first polarizing device (210) having a first polarization direction, the polarization beam splitter (770) having a fourth polarization direction (S-polarization), the first polarization direction and the fourth polarization direction being perpendicular to each other, the first quarter wave plate (230) having a first optical axis direction, the second quarter wave plate (240) having a second optical axis direction, wherein the first optical axis direction and the second optical axis direction are both disposed between the first polarization direction and the fourth polarization direction for enhancing an interference efficiency and an image quality of an orthogonal polarization spectral absorption image.

8. The optical sectioning apparatus using advanced optical interference microscopy according to claim 7, wherein the first white light beam (10) has a wavelength ranging from 400 nm to 800 nm and includes a blue narrowband having a wavelength ranging from 400 nm to 480 nm and a yellow or orange broadband having a wavelength ranging from 480 nm to 800 nm, the shortwave light beam (20) having a wavelength ranging from 350 nm to 410 nm, the first quarter wave plate (230), the second quarter wave plate (240), the first beam splitter (220), the second beam splitter (710), the first polarizer (210), and the polarization beam splitter (770) being configured to operate with a wavelength ranging from 400 nm to 800 nm, the dichroic beam splitter (260) having a cut-off wavelength range between 360 nm and 420 nm, and the long pass filter (740) having a cut-off wavelength range between 460 nm and 510 nm.

9. The optical sectioning apparatus using advanced optical interference microscopy according to any of claims 1 to 3, further comprising an information processing unit for performing an image processing procedure.

10. The optical sectioning apparatus using advanced optical interference microscopy according to claim 9, wherein the reference end unit (600) further has an axial motion platform (640), and the carrier unit (400) further includes a 3-D motion platform (430), so that by using the axial motion platform (640) to move the second objective lens (620) and the reflective mirror (630) of the reference end unit (600), using the 3-D motion platform (430) to move the sample (410) dyed with a fluorescent agent, and adjusting the optical path delay unit (610), the information processing apparatus can derive a 3-D image of the sample (410) accordingly.

11. The optical sectioning apparatus using advanced optical interference microscopy according to claim 9, wherein the carrier unit (400) further has a 3-D motion platform (430) and a second white light source unit (420), wherein the second white light source unit (420) includes a white LED, a white halogen lamp or a tungsten lamp, and the second white light source unit (420) is configured to provide the first objective lens (300) with a suitable penetration brightness, so that when a H&E slice is placed on the carrier unit (400), the 3-D motion platform (430) will move the H&E slice and the second white light source (420) will generate a second white light beam (40) to illuminate the H&E slice to enable the information processing unit to execute the image processing procedure according to image data sensed by the sensing unit (700) to generate an H&E image.
